(19) **Europäisches Patentamt / European Patent Office / Office européen des brevets**

(11) **EP 4 623 816 A1**

(12) **EUROPEAN PATENT APPLICATION**
published in accordance with Art. 153(4) EPC

(43) Date of publication:
01.10.2025 Bulletin 2025/40

(21) Application number: 23894763.4

(22) Date of filing: 12.09.2023

(51) International Patent Classification (IPC):
*A61B 5/021* (2006.01)   *A61B 5/024* (2006.01)
*A61B 5/00* (2006.01)   *G16H 50/20* (2018.01)

(52) Cooperative Patent Classification (CPC):
A61B 5/00; A61B 5/021; A61B 5/024; G16H 50/20

(86) International application number:
PCT/KR2023/013662

(87) International publication number:
WO 2024/111831 (30.05.2024 Gazette 2024/22)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 23.11.2022 KR 20220157952

(71) Applicant: **Sky Labs Inc.**
**Gyeonggi-do 13486 (KR)**

(72) Inventors:
• **LEE, Min Hyung**
**Bucheon-si Gyeonggi-do 14600 (KR)**
• **KIM, Chang Hyun**
**Seoul 05372 (KR)**
• **CHOI, Chang Woo**
**Uiwang-si Gyeonggi-do 16000 (KR)**
• **LEE, Byung Hwan**
**Yongin-si Gyeonggi-do 16981 (KR)**

(74) Representative: **Gallo, Luca et al**
**Gallo & Partners S.r.l.**
**Via Rezzonico, 6**
**35131 Padova (IT)**

(54) **METHOD AND SYSTEM FOR TRAINING BLOOD PRESSURE ESTIMATION MODEL BY USING PHOTOPLETHYSMOGRAPHY SIGNAL**

(57)　An embodiment of the present invention relates to a method and system for learning a blood pressure estimation model using photoplethysmography signal (PPG). The blood pressure estimation model learning method includes the steps of: preprocessing raw data including blood pressure signal data as well as PPG data collected from subjects; and constructing a blood pressure estimation model based on the preprocessed blood pressure signal data and PPG data. The raw data includes blood pressure signal data, a variance level of which is greater than or equal to a predetermined value.

[FIG. 2]

**Description**

[Field of Invention]

An embodiment of the present invention relates to a method and system for learning a blood pressure estimation model using an optical volume change signal. More particularly, the present invention relates to a method and system for learning a blood pressure estimation model, which can estimate blood pressure with high reliability even in the case where a variation in blood pressure of the subject is high, by learning data having a large extent of deviation between subjects and data having a large extent of deviation within the subject.

[Background of Invention]

Recently, the prevalence of high blood pressure is increasing due to the aging of society and the westernization of lifestyle and diet owing to the development of medical technology. High blood pressure ('hypertension') is a major indicator of kidney disease and serious cardiovascular disease and is one of the most dangerous causes of death, therefore, treatment and management of this condition are essential.

The most important thing to prevent, recognize, and treat hypertension is to continuously measure blood pressure during daily life, but this is not kept well.

A typical method of monitoring blood pressure has been to wear a cuff and measure blood pressure from changes in pressure injected into the cuff. The method using a cuff has the disadvantages of causing discomfort due to pressure during the measurement and that it is not easy to actually carry a blood pressure measuring device because a cuff is necessarily included therein even if you purchase a portable product. The method using a cuff is not suitable for real-time monitoring of blood pressure due to disadvantages in both convenience and portability. Accordingly, research on measuring blood pressure without restraints of cuff is actively underway.

In particular, research using PPG (Photoplethysmography) optical sensors has been increasing recently, because vascular elasticity information can be found using characteristic values such as percussion wave, tidal wave, etc. in the PPG signal. Since vascular elasticity information has a very high correlation with blood pressure, blood pressure can be estimated using this information.

Another reason why there are many attempts to measure blood pressure through PPG signals is because the PPG optical sensor has great significance as a personal cuff-less blood pressure measurement device. Recently, PPG optical sensors are commonly installed in most wearable devices such as smart bands and watches. This means that most wearable devices can have blood pressure measurement functions without the need of adding new sensors so far as a desired program is simply installed. If blood pressure can be measured through the PPG optical sensor of a wearable device, existing blood pressure measurement methods may be greatly improved in terms of portability and convenience. In terms of convenience, wearable devices such as smart bands and smart watches have the advantage that the wearer does not feel much discomfort when worn on the wrist. In summary, using PPG signal may estimate blood pressure with high accuracy, and the convenience and portability problems of existing devices can be solved by applying it to any wearable device.

In recent years, attempts to estimate blood pressure from PPG signals using artificial intelligence technology are actively underway since there is a correlation between PPG data and blood pressure that has not been fully identified. There have been attempts to identify unidentified correlations through artificial intelligence. However, some of the existing learning-based systems had limitations in not correctly estimating highly variable blood pressure of a subject since they were modeled and tested in a 'subject dependent' manner.

[Summary of Invention]

[Technical Problem to be Solved]

The present invention was created in the background described above, and seeks to provide a method for learning a highly reliable blood pressure estimation model even for highly variable blood pressure using a convolutional neural network.

The problem to be solved by the present invention is not limited to the problem(s) mentioned above, and other problem(s) not mentioned herein will be clearly understood by those skilled in the art from the description below.

[Technical Solution]

To this end, according to an embodiment of the present invention, a blood pressure estimation model learning method using a photoplethysmography signal (PPG), in which each step is performed by a blood pressure estimation model learning system using artificial intelligence, may include: preprocessing raw data including optical volume change signal (that is, PPG) data and blood pressure signal data collected from subjects; and constructing a blood pressure estimation model based on the preprocessed PPG data and blood pressure signal data, wherein the raw data may include blood pressure signal data with a variance level greater than or equal to a preset value.

A blood pressure estimation model learning system using a photoplethysmography signal (PPG) according to another embodiment of the present invention may include a preprocessor to preprocess raw data including PPG data and blood pressure signal data collected from subjects, and a model construction unit to construct a blood pressure estimation model based on the preprocessed PPG data and blood pressure signal data, wherein the raw data may include blood pressure signal data with a variance level greater than or equal to a preset value.

[Effect of Invention]

According to the present invention, a learning model is constructed using blood pressure signal data with a variance level greater than or equal to a preset value, thus making it possible to build a highly reliable blood pressure estimation model even in various environments where a subject's blood pressure is not stable.

Further, since the blood pressure estimation model according to an embodiment of the present invention is independent of the subject, it is possible to estimate the subject's highly variable blood pressure with high reliability.

Effects of the present invention are not limited to the effects mentioned above, and other effects not mentioned herein will be clearly understood by those skilled in the art from the description below.

[Brief Description of Drawings]

FIG. 1 is a diagram schematically showing the configuration of a blood pressure estimation model learning system using PPG according to an embodiment of the present invention.

FIG. 2 is a conceptual diagram illustrating the blood pressure estimation model learning system using PPG according to an embodiment of the present invention.

FIG. 3 is a block diagram for explaining the configuration of a preprocessor according to an embodiment of the present invention.

FIG. 4 is a conceptual diagram for explaining a preprocessing method according to an embodiment of the present invention.

FIG. 5 is a diagram for explaining the standard deviation of subject-calibration centering according to an embodiment of the present invention.

FIG. 6 is a conceptual diagram for explaining a blood pressure estimation model according to an embodiment of the present invention.

FIG. 7 is a flowchart illustrating a method of constructing a blood pressure estimation model according to an embodiment of the present invention.

FIG. 8 is a block diagram for explaining the configuration of a blood pressure estimation device according to an embodiment of the present invention.

[Concrete Description of Preferred Embodiments of Invention]

Hereinafter, embodiments according to the present invention will be described in detail with reference to the attached drawings. The configuration of the present invention and its operational effects will be clearly understood through the detailed description below. Prior to the detailed description of the present invention, it is noted that the same components will be indicated by the same reference numerals as much as possible even if they are shown in different drawings, and detailed descriptions of known components will be omitted if it is judged that they may obscure the gist of the present invention.

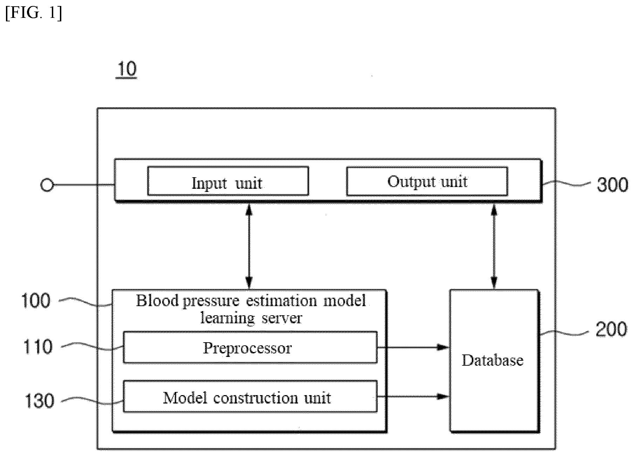

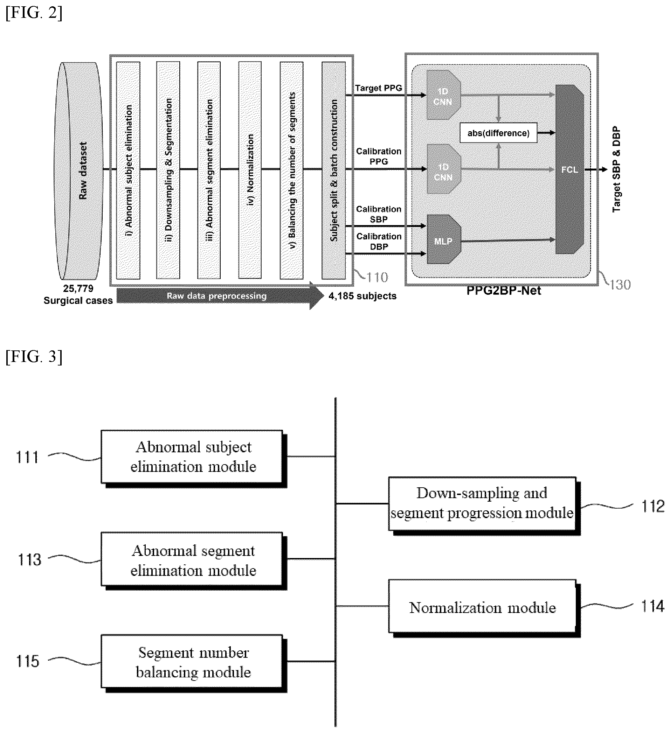

FIG. 1 is a diagram schematically showing the configuration of a blood pressure estimation model learning system using photoplethysmography signal (hereinafter, referred to as PPG) according to an embodiment of the present invention, while FIG. 2 is a conceptual diagram illustrating the blood pressure estimation model learning system using PPG according to an embodiment of the present invention.

As shown in FIGS. 1 and 2, the blood pressure estimation model learning system 10 using PPG according to an embodiment of the present invention may include a blood pressure estimation model learning server 100, a database 200, and an input/output device 300.

The blood pressure estimation model learning server 100 may preprocess raw data including PPG data and blood pressure signal data measured from subjects, and learn the preprocessed data to thus learn a model for estimating blood pressure from the user's PPG.

The raw data is divided into training data and verification data on a subject-independent basis.

The blood pressure estimation model learning server 100 may include a preprocessor 110 and a model construction unit 130.

The preprocessor 110 may preprocess the subject's PPG data and blood pressure signal data previously stored in the database 200. Preprocessing is performed to increase a learning time and learning effect of the learning results, and to improve the reliability of the learning results.

In one embodiment, the preprocessor 110 removes abnormal data. In order to remove abnormal data, additional information of the subject must also be collected when collecting raw data. Additional information of the subject may include, for example, weight, height, age, pregnancy status, surgery time log, and electrocardiogram.

In one embodiment, the preprocessor 110 may perform the following five steps for data preprocessing.

First, abnormal subjects are removed from raw data.

Second, down-sampling and segmentation of raw data is performed.

Third, abnormal segments are removed from raw data.

Fourth, normalization of raw data is performed.

Fifth, the number of segments is balanced.

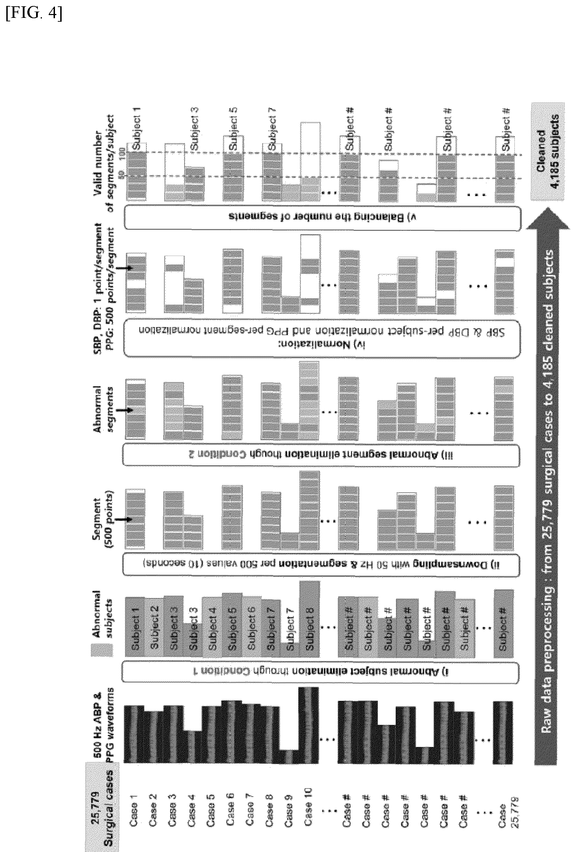

The specific preprocessing method will be described with reference to FIGS. 3 and 4.

The model construction unit 130 learns how to estimate blood pressure based on preprocessed data.

The model used to learn the blood pressure estimation model may include two 1D-CNNs. Herein, one 1D-CNN extracts temporal features of PPG collected as raw data, and another 1D-CNN extracts morphological features from PPG differences. The CNN layer consists of multiple kernels and uses 'rectified linear unit (ReLU)' as an activation function.

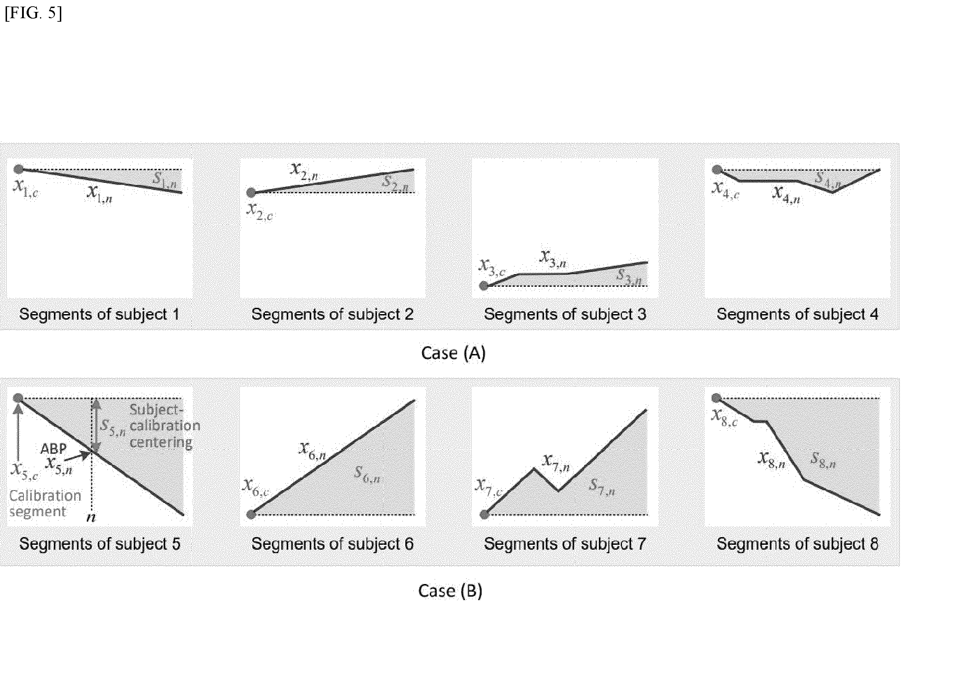

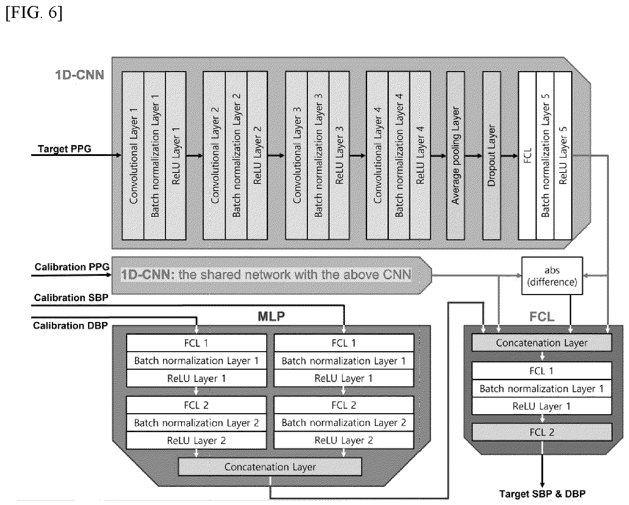

A structure and learning method of the model used to learn the blood pressure estimation model will be described with reference to FIGS. 5 and 6.

The database 200 stores or databases information, programs, etc. necessary for the blood pressure estimation model learning server 100 to construct or learn a blood pressure estimation model using PPG. For example, the database 200 may store raw data for constructing a blood pressure estimation model, or store data preprocessed by the blood pressure estimation model learning server 100. Further, intermediate data generated by the blood pressure estimation model learning server 100 to construct a blood pressure estimation model and the constructed blood pressure estimation model may be stored. This database 200 may include at least one storage, DB server, or file server.

Meanwhile, raw data according to one embodiment includes PPG data and blood pressure signal data collected from subjects.

Raw data includes blood pressure signal data with a variance level greater than or equal to a set value. Herein, the variance level is defined by a standard deviation (SDS) of subject-calibration centering.

The standard deviation (SDS) of subject-calibration centering for blood pressure signal data is calculated by Equation 1.

[Equation 1]

$$SDS = \sqrt{\frac{1}{\sum_i N_i - 1} \sum_i \sum_{n=1}^{N_i} \left( s_{i,n} - \bar{s} \right)^2}$$

At this time, $N_i$ is the number of segments of subject i, and $S_{i,n}$ is calculated by Equation 2, and $\bar{s}$ is calculated by Equation 3,

[Equation 2]

$$s_{i,n} = x_{i,n} - x_{i,c}$$

In this regard, $x_{i,n}$ is artery blood pressure (ABP) of the nth segment of subject i, and $x_{i,c}$ is ABP used for calibration of subject i. The artery blood pressure (ABP) is a hemodynamic index that guides clinicians in providing therapeutic interventions, which is a pressure of blood on the walls of arteries and is mainly measured in the brachial artery. It is known that systolic blood pressure of 120 mmHg and diastolic blood pressure of 80 mmHg or less are considered

normal.

[Equation 3]

$$\bar{s} = \frac{1}{\sum_i N_i} \sum_i \sum_{n=1}^{N_i} s_{i,n}$$

In one embodiment, using blood pressure signal data with a variance level greater than or equal to a preset value as raw data may construct a highly reliable blood pressure estimation model even in various environments where the blood pressure of a subject is unstable.

The input/output device 300 may include an input unit and an output unit. The input unit includes input means capable of being operated by the user, such as inputting and selecting data. The input means may include a general keypad, mouse, etc., and if the input unit is provided as a touch screen enabling touch-input, it may be provided integrally with the output unit.

The output unit is configured to display various information related to the operation of blood pressure estimation model learning under the control of the blood pressure estimation model learning server 100.

This output unit may be implemented as, for example, a liquid crystal display (LCD), a light emitting diode (LED), an organic light emitting diode (OLED), a projector, or other display devices that are currently available, have been available in the past, or will be available in the future. The output unit may display, for example, an interface page for providing information or a result page for providing information.

FIG. 3 is a block diagram for explaining the configuration of a preprocessor according to an embodiment of the present invention, while FIG. 4 is a conceptual diagram for explaining a preprocessing method according to an embodiment of the present invention.

Referring to FIGS. 3 and 4, the preprocessor 110 includes an abnormal subject elimination module 111, a down-sampling and segment progression module 112, an abnormal segment elimination module 113, a normalization module 114, and a balance adjustment module 115 for the number of segments (hereinafter, referred to as 'segment number balancing module').

The abnormal subject elimination module 111 removes abnormal subjects for data preprocessing.

The abnormal subject elimination module 111 removes abnormal and duplicate data from the collected data. At this time, abnormal data, for example, abnormal data of subjects in exceptional conditions and almost identical ABP and PPG data may be removed.

In this regard, the criteria for exceptional conditions (C1-1 C1-2, C1-3) may include the following three cases.

A first criterion C1-1 of exceptional conditions may include the subject's weight, height, and pregnancy status. For example, a normal subject according to the first criterion may be persons with $10 \leq$ weight $\leq 100$ kg, $100 \leq$ height $\leq 200$ cm, $18 \leq$ age $\leq 100$ years, and who are not pregnant. In other words, data from subjects that deviate from the standards for normal subjects can be removed as abnormal data.

A second criterion C1-2 of exceptional conditions is based on the subject's essential information, and includes surgery time log, electrocardiogram, PPG, ART-SBP (systolic blood pressure), ART-DBP (diastolic blood pressure) and ART-MBP (mean blood pressure) may be included.

A third criterion C1-3 of exceptional conditions may be noise. The abnormal subject elimination module 111 may remove PPG or ABP waveforms containing noise.

That is, the abnormal subject elimination module 111 removes data of subjects that violate any of the above-mentioned exceptional condition criteria (C1-1, C1-2, and C1-3).

Next, the down-sampling and segmentation module 112 performs down-sampling and segmentation on the raw data.

The down-sampling and segment progression module 112 may down-sample the ABP and PPG data sampled at, for example, 500 Hz to a preset first standard, for example, 50Hz, and then divides them into several segments each being composed of a preset second standard. At this time, the preset second standard may be, for example, 500 points (i.e., 10 seconds of data per segment). In another modified embodiment, the segments may be split into 8-second lengths to design ANN16 and LRCN24, or the segments may be split into 10-second lengths to design SVR.

The abnormal segment elimination module 113 removes abnormal segments from the divided segments. The abnormal segments may include segments with invalid pulse rates, abnormal SBP/DBP fluctuations, or irregular pulses. The ABP segment of normal SBP is $70 \leq$ average SBP $\leq 180$ mmHg, so that segments out of this range can be removed. The normalization module 114 performs normalization.

A-line SBP and DBP are composed of the average values of peak systolic and end-diastolic pressures at each A-line pulse. SBP and DBP values are normalized to the mean and SD of the entire training set.

The segment number balancing module 115 may balance the number of segments.

In order to balance the number of segments, the segment number balancing module 115 may remove normalized subjects whose segments are less than a preset minimum number. Additionally, the segment number balancing module 115 randomly selects only 100 segments when the subject has more than a preset maximum number of segments. Therefore, each subject may contain more than the minimum number but less than the maximum number of segments. Herein, the minimum number may be 50 and the maximum number may be 100, but are not limited thereto. By balancing the number of segments, every subject can have fair effects on training and verification.

FIG. 5 is a diagram for explaining the standard deviation of subject-calibration centering according to an embodiment of the present invention.

Since the blood pressure estimation learning model learns PPG features that are dynamically changed according to BP changes for new subjects, PPG-based BP estimation accuracy improves with increase in the number of subjects used in modeling.

In one embodiment, if PPG samples from the same subject are used for training and test data, the model may overfit the subject. Accordingly, in one embodiment, a subject-independent data set is used. In other words, the data set used for training and the data set used for testing are composed of different subjects. Further, a holdout method may be used for non-exclusive cross-verification and testing. Since the holdout method is a conventionally known method, detailed description thereof is omitted.

On the other hand, if within-subject BP variation is low, accuracy performance may be overqualified.

Referring to FIG. 5, Case A represents an example showing high BP variation between subjects but small variation within subjects. On the other hand, Case B represents an example of not only high BP variation between subjects, but also high variation within subjects.

One embodiment of the invention includes data with high BP variation within subjects, such as Case A as well as Case B.

FIG. 6 is a conceptual diagram for explaining a blood pressure estimation model according to an embodiment of the present invention.

Referring to FIG. 6, the model construction unit (130 in FIG. 1) may learn using a learning model including two 1D-CNNs, one multilayer perceptron (MLP), and one fully connected layer (FCL).

The two 1D-CNNs have the same structure and parameters as a main feature extraction network. A target PPG is input to one 1D-CNN, and time series features of the PPG waveform are extracted using multiple filters. In another $1\times500$ 1D-CNN, calibration PPG is input for training, and various features are extracted from the calibration PPG waveform using multiple filters.

In this regard, 1D-CNN includes four groups of hidden CNN layers, an average pooling layer and a drop outlayer. Each hidden CNN layer group consists of one convolution layer, a batch normalization layer, and a rectification linear unit (ReLU) layer. Batch normalization between the convolutional layer and the ReLU layer normalizes the hidden layer input and solves problems caused by changes in the input distribution. ReLU layers are used at the end of each hidden layer for faster and better learning.

After four groups of hidden CNN layers, the waveforms are sampled through an average pooling layer, which in turn reduces the complexity of the network by retaining the essential information of the features. 30% of the output data from the average pooling layer is dropped out (e.g. set to 0) in the dropout layer by randomly removing 30% of the neurons during training. If dropout is set to 0, a hyperparameter dropout rate will be 0.3. Dropout reduces overfitting and improves generalization by preventing meaningless actions from relying heavily on specific inputs.

After the dropout layer, each batch passes FCL with 8 units and is normalized in the batch normalization layer such that the mean and variance become 0 and 1, respectively, thereby improving convergence speed and learning performance.

The two 1D-CNN output sequences and absolute difference therebetween are provided as input to the final FCL module and activated by the ReLU function.

MLP is used to support feature extraction for supervised learning from A-line SBP and DBP values. Calibration SBP and calibration DBP values are input to the MLP and provided to each of the two FCLs. After each FCL, a batch normalization layer and a ReLU layer are disposed. The output of each ReLU layer is input to the connection layer. The output of the connection layer is input to the FCL and the final target SBP and DBP are output.

Features output from two 1D-CNNs, differences between them, and MLP are connected. A single output sequence from the connection layer is provided to the FCL, where the batch normalization layer and ReLU layer are deployed. The output of the ReLU layer generates target SBP and DBP through different FCLs.

The blood pressure estimation model according to one embodiment acquired data on 4185 subjects from 25779 surgical cases in the preprocessing process. At this time, 80% was used as training data, and 20% was used for holdout verification to evaluate the model's performance. Further, in order to prevent model overfitting, 10% of the training data was randomly selected and used as verification data. In addition, the feasibility of applying the proposed model to medical devices was verified using the BHS and AAMI standards, which are blood pressure monitor certification standards.

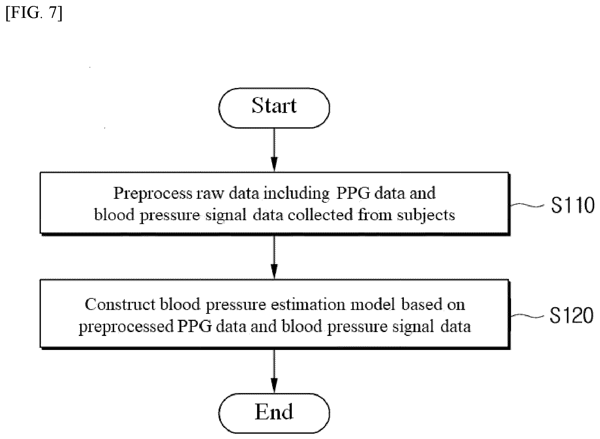

FIG. 7 is a flowchart illustrating a method of constructing a blood pressure estimation model according to an embodiment of the present invention.

The method of constructing a blood pressure estimation model described with reference to FIG. 7 may be performed by the blood pressure estimation model learning system described with reference to FIGS. 1 to 6.

In step S110, raw data including PPG data and blood pressure signal data collected from subjects are preprocessed. The raw data includes blood pressure signal data with a variance level greater than or equal to a preset value. The variance level is the standard deviation of subject-calibration centering.

The standard deviation (SDS) of subject-calibration centering can be calculated using Equations 1, 2, and 3 described above.

To preprocess raw data, firstly, abnormal subjects that do not meet predefined conditions are removed from the raw data. Next, the raw data is down-sampled and divided into multiple segments. Afterwards, predefined abnormal segments are removed from the divided segments. Next, the raw data from which abnormal segments are removed is normalized. Finally, normalized subjects less than the preset minimum number are removed, while subjects greater than the preset maximum number are randomly selected to balance the number of segments.

A blood pressure estimation model may be constructed based on the PPG data and blood pressure signal data preprocessed in step S 120.

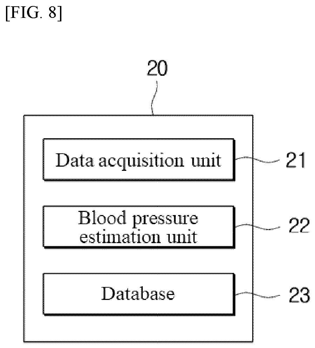

FIG. 8 is a block diagram for explaining the configuration of a blood pressure estimation device according to an embodiment of the present invention.

Referring to FIG. 8, the blood pressure estimation device 20 according to an embodiment of the present invention may estimate the patient's abnormal blood pressure (e.g., low blood pressure or high blood pressure) using the acquired PPG data. For example, the blood pressure estimation device 20 can estimate the patient's blood pressure using a blood pressure estimation model constructed with reference to FIGS. 1 to 6 using PPG data as an input value.

In one embodiment, the blood pressure estimation device 20 may be a surgical monitoring server, a computer, or a medical device, and a dedicated program that can set a blood pressure estimation model and a blood pressure prediction method may be installed. For example, the blood pressure estimation device 20 may include a data acquisition unit 21 capable of acquiring PPG data, a blood pressure estimation unit 22 for estimating blood pressure using the acquired data and a blood pressure estimation model, and a database 23 capable of converting the blood pressure estimation model, the blood pressure estimates, etc. into big data and storing the same.

A data acquisition unit 21 may acquire PPG data by performing A/D conversion on the PPG acquired from patients. For example, the data acquisition unit 21 may receive data from an external device, receive the data as input from a user (e.g., medical staff), or receive PPG signal from a PPG sensor. PPG data is acquired from the waveform of the PPG. It may include characteristic values such as the time on which each waveform of the PPG is maintained, the interval between waveforms, the amplitude of each waveform, and kurtosis. Further, PPG data may include representative values such as average, maximum, or minimum values in addition to waveforms.

The data acquisition unit 21 may sample each PPG data as needed.

The blood pressure estimation unit 22 inputs the PPG data obtained from the data acquisition unit 21 to the blood pressure estimation model constructed with reference to FIGS. 1 to 7, and outputs a blood pressure estimate.

Meanwhile, although not shown in the drawing, the blood pressure estimation device 20 may further include an electrocardiogram sensor.

In this regard, it will be understood that each block in the processing flow diagrams and combinations of the flow diagram diagrams can be performed by computer program instructions. These computer program instructions may be loaded in a processor of a general-purpose computer, special-purpose computer, or other programmable data processing equipment, so that the instructions executed through the processor of the computer or other programmable data processing equipment would create the means for performing functions described in the flow diagram block(s). These computer program instructions may also be stored in computer-usable or computer-readable memory that can be directed to a computer or other programmable data processing equipment to perform a function in a specific manner, so that the instructions stored in the computer-usable or computer-readable memory may also produce manufacturing items provided with instruction means that perform the functions described in the flow diagram block(s). Computer program instructions can also be loaded on a computer or other programmable data processing equipment so that a series of operational steps are executed on the computer or other programmable data processing equipment to create a process performed by the computer, whereby instructions to implement the computer or other programmable data processing equipment may also provide steps for performing the functions described in the flow diagram block(s).

Further, each block may represent a portion of module, segment, or code that includes one or more executable instructions for performing specified logical function(s). Further, it should be noted that, in some alternative embodiments, the functions mentioned in the blocks may possibly occur out of order. For example, two blocks shown in succession may be performed substantially at the same time, otherwise, in reverse order depending on the corresponding function.

Herein, the term '~unit' used in this embodiment refers to software or hardware components such as FPGA or

# EP 4 623 816 A1

ASIC, and the '~unit' performs certain role. However, '~unit' is not limited to software or hardware. The '~ unit' may be configured to reside in an addressable storage medium or may be configured to reproduce on one or more processors. Therefore, for example, '~ unit' refers to components such as software components, object-oriented software components, class components and task components, processes, functions, properties, procedures, subroutines, segments of program code, drivers, firmware, microcode, circuitry, data, databases, data structures, tables, arrays, and variables. The functions provided within the components and 'units' may be combined into a smaller number of components and 'units', or may be further separated into additional components and 'units'. Further, the components and 'units' may be implemented to regenerate one or more CPUs within the device or security multimedia card.

Those skilled in the art to which this disclosure pertains will understand that the disclosure can be implemented in other specific forms without changing its technical idea or essential features. Therefore, the embodiments described above should be understood to be illustrative in all respects and not restrictive. The scope of the present disclosure is indicated by the scope of the claims described later rather than the detailed description above, however, it should be interpreted that all changes or modifications derived from the meaning, scope, and equivalent concept of the claims are included in the scope of the present disclosure.

Meanwhile, the specification and drawings disclose preferred embodiments of the present disclosure, and although specific terms are used, they are used only in a general sense to easily explain the technical content of the present disclosure and aid understanding of the invention, however, it is not intended to limit the scope of the disclosure. In addition to the embodiments disclosed herein, it is obvious to those skilled in the art that other modifications based on the technical idea of the present disclosure can be implemented.

[Description of Reference Numeral]

10: Blood pressure estimation model learning system
100: Blood pressure estimation model learning server
110: Preprocessor
111: Abnormal subject elimination module
112: Down-sampling and segment progression module
113: Abnormal segment elimination module
114: Normalization module
115: Segment number balancing module
130: Model construction unit
200: Database
300: Input/output unit

## Claims

1. A blood pressure estimation model learning method using photoplethysmography signal (PPG), in which each step is performed by a blood pressure estimation model learning system using artificial intelligence, the method comprising:

   preprocessing raw data including optical volume change signal (that is, PPG) data and blood pressure signal data collected from subjects; and
   constructing a blood pressure estimation model based on the preprocessed PPG data and blood pressure signal data,
   wherein the raw data includes blood pressure signal data with a variance level greater than or equal to a preset value.

2. The method according to claim 1, wherein the variance level is a standard deviation (SDS) of subject-calibration centering.

3. The method according to claim 2, wherein the standard deviation (SDS) of subject-calibration centering is calculated by Equation 1,

8

[Equation 1]

$$SDS = \sqrt{\frac{1}{\sum_i N_i - 1} \sum_i \sum_{n=1}^{N_i} \left(s_{i,n} - \bar{s}\right)^2}$$

wherein $N_i$ is the number of segments of subject i, and $S_{i,n}$ is calculated by Equation 2, and $\bar{s}$ is calculated by Equation 3,

[Equation 2]

$$s_{i,n} = x_{i,n} - x_{i,c}$$

wherein, $x_{i,n}$ is artery blood pressure (ABP) of the nth segment of subject i, and $x_{i,c}$ is ABP used for calibration of subject i, and

[Equation 3]

$$\bar{s} = \frac{1}{\sum_i N_i} \sum_i \sum_{n=1}^{N_i} s_{i,n}$$

4. The method according to claim 2, wherein the preprocessing step of the raw data comprises:

   removing abnormal subjects not meeting predefined conditions from the raw data;
   down-sampling and dividing the raw data into a plurality of segments;
   removing a predefined abnormal segment from the divided segments;
   normalizing the raw data from which abnormal segments have been removed; and
   balancing the number of segments by removing normalized subjects less than a preset minimum number and randomly selecting the maximum number of segments for subjects greater than or equal to a preset maximum number.

5. The method according to claim 1, wherein the step of constructing the blood pressure estimation model comprises: using a neural network including two 1D-CNNs, one MLP (multilayer perceptron), and one FCL (fully connected layer).

6. The method according to claim 5, wherein the 1D-CNN is stacked with 4 CNNs, an average pooling layer, FCL, batch layer 5 and ReLU layer 5.

7. The method according to claim 1, wherein the raw data is divided into training data and verification data based on subjects (subject-independent).

8. A blood pressure estimation model learning system using photoplethysmography signal (PPG), comprising:

   a preprocessor that preprocesses raw data including PPG data and blood pressure signal data collected from subjects; and
   a model construction unit that constructs a blood pressure estimation model based on the preprocessed PPG data and blood pressure signal data,
   wherein the raw data includes blood pressure signal data with a variance level greater than or equal to a preset value.

9. The system according to claim 8, wherein the variance level is the standard deviation (SDS) of subject-calibration centering.

10. The system according to claim 9, wherein the SDS of subject-calibration centering is calculated by: Equation 1,

[Equation 1]

$$SDS = \sqrt{\frac{1}{\sum_i N_i - 1} \sum_i \sum_{n=1}^{N_i} \left(s_{i,n} - \bar{s}\right)^2}$$

wherein Ni is the number of segments of subject i, and $S_{,i,n}$ is calculated by Equation 2, and $\bar{s}$ is calculated by Equation 3,

[Equation 2]

$$s_{i,n} = x_{i,n} - x_{i,c}$$

wherein, $x_{i,n}$ is artery blood pressure (ABP) of the nth segment of subject i, and $x_{i,c}$ is ABP used for calibration of subject i, and

[Equation 3]

$$\bar{s} = \frac{1}{\sum_i N_i} \sum_i \sum_{n=1}^{N_i} s_{i,n}$$

[FIG. 1]

10

[FIG. 2]

[FIG. 3]

[FIG. 4]

[FIG. 5]

[FIG. 6]

[FIG. 7]

Start

Preprocess raw data including PPG data and blood pressure signal data collected from subjects — S110

Construct blood pressure estimation model based on preprocessed PPG data and blood pressure signal data — S120

End

[FIG. 8]

## INTERNATIONAL SEARCH REPORT

| | International application No. |
|---|---|
| | **PCT/KR2023/013662** |

| **A.** | **CLASSIFICATION OF SUBJECT MATTER** |
|---|---|

**A61B 5/021**(2006.01)i; **A61B 5/024**(2006.01)i; **A61B 5/00**(2006.01)i; **G16H 50/20**(2018.01)i

According to International Patent Classification (IPC) or to both national classification and IPC

| **B.** | **FIELDS SEARCHED** |
|---|---|

Minimum documentation searched (classification system followed by classification symbols)

A61B 5/021(2006.01); A61B 5/00(2006.01); A61B 5/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 광용적맥파 (Photoplethysmography), 혈압 (blood pressure), 학습 (learning), 추정 (estimation), 모델 (model), 분산 (dispersion)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| Y | JP 2022-148276 A (SHINSHU UNIV) 06 October 2022 (2022-10-06)<br>    See paragraph [0063]; and claim 1. | 1,7,8 |
| A | | 2-6,9,10 |
| Y | CANO et al. The relevance of calibration in machine learning-based hypertension risk assessment combining photoplethysmography and electrocardiography. Biosensors. 01 May 2022, vol. 12, no. 5: 289, pp.:1-14.<br>    See abstract; and page 7. | 1,7,8 |
| A | US 2020-0229716 A1 (KONINKLIJKE PHILIPS N.V.) 23 July 2020 (2020-07-23)<br>    See claims 1-11. | 1-10 |
| A | KR 10-2019-0056858 A (GACHON UNIVERSITY OF INDUSTRY-ACADEMIC COOPERATION FOUNDATION et al.) 27 May 2019 (2019-05-27)<br>    See entire document. | 1-10 |

✓ Further documents are listed in the continuation of Box C.　　✓ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **14 December 2023** | **14 December 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/013662** |

**C.    DOCUMENTS CONSIDERED TO BE RELEVANT**

| Category* | Citation of document, with indication, where appropriate, of the relevant passages | Relevant to claim No. |
| --- | --- | --- |
| A | KR 10-2022-0129282 A (ELECTRONICS AND TELECOMMUNICATIONS RESEARCH INSTITUTE) 23 September 2022 (2022-09-23)<br>  See entire document. | 1-10 |

Form PCT/ISA/210 (second sheet) (July 2022)

**INTERNATIONAL SEARCH REPORT**
Information on patent family members

International application No.

**PCT/KR2023/013662**

| Patent document cited in search report | | | Publication date (day/month/year) | Patent family member(s) | | | Publication date (day/month/year) |
|---|---|---|---|---|---|---|---|
| JP | 2022-148276 | A | 06 October 2022 | None | | | |
| US | 2020-0229716 | A1 | 23 July 2020 | CN | 111065323 | A | 24 April 2020 |
| | | | | CN | 111065323 | B | 28 April 2023 |
| | | | | EP | 3430991 | A1 | 23 January 2019 |
| | | | | EP | 3654837 | A1 | 27 May 2020 |
| | | | | JP | 2020-527073 | A | 03 September 2020 |
| | | | | JP | 7269916 | B2 | 09 May 2023 |
| | | | | US | 11583191 | B2 | 21 February 2023 |
| | | | | WO | 2019-016405 | A1 | 24 January 2019 |
| KR | 10-2019-0056858 | A | 27 May 2019 | KR | 10-2042700 | B1 | 08 November 2019 |
| KR | 10-2022-0129282 | A | 23 September 2022 | US | 2022-0296172 | A1 | 22 September 2022 |

Form PCT/ISA/210 (patent family annex) (July 2022)